(19) Europäisches Patentamt
European Patent Office
Office européen des brevets

(11) **EP 3 467 531 A1**

(12) **EUROPÄISCHE PATENTANMELDUNG**

(43) Veröffentlichungstag:
**10.04.2019 Patentblatt 2019/15**

(51) Int Cl.:
**G01R 33/36** *(2006.01)*     **G01R 33/565** *(2006.01)*
**G01R 33/422** *(2006.01)*

(21) Anmeldenummer: **17194969.6**

(22) Anmeldetag: **05.10.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare GmbH**
**91052 Erlangen (DE)**

(72) Erfinder: **Biber, Stephan**
**91056 Erlangen (DE)**

(54) **MAGNETRESONANZTOMOGRAPH MIT AKTIVER STÖRUNTERDRÜCKUNG UND VERFAHREN ZUR STÖRUNTERDRÜCKUNG IN EINEM MAGNETRESONANZTOMOGRAPHEN**

(57)     Die Erfindung betrifft einen Magnetresonanztomograph en sowie ein Verfahren zum Betrieb des Magnetresonanztomographen. Der Magnetresonanztomograph weist eine erste Empfangsantenne zum Empfang eines Magnetresonanzsignals aus einem Patienten (100) in einem Patiententunnel, eine zweite Empfangsantenne zum Empfang eines Signals mit der Larmorfrequenz des Magnetresonanzsignals, und einen Empfänger auf. Die zweite Empfangsantenne ist außerhalb oder in der Nähe einer Öffnung des Patiententunnels angeordnet. Der Empfänger steht in Signalverbindung mit der ersten Empfangsantenne und der zweiten Empfangsantenne und ist ausgelegt, ein mit der zweiten Empfangsantenne empfangenes Störsignal in einem von der ersten Empfangsantenne empfangenen Magnetresonanzsignal zu unterdrücken.

FIG 2

**Beschreibung**

Die Erfindung betrifft ein Verfahren zur aktiven Störunterdrückung in einem Magnetresonanztomographen sowie einen Magnetresonanztomographen mit einem Empfänger.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden. Die Empfangsantennen können auch in einer Patientenliege verbaut sein.

Magnetresonanztomographen erfordern in zweierlei Hinsicht eine Hochfrequenzabschirmung. Zum einen werden zur Anregung der Kernspins Hochfrequenzimpulse mit Leistungen im Kilowattbereich erzeugt, die nur teilweise im Patienten absorbiert werden. Radiowellen, die die Patientendurchführung verlassen, werden in den Raum abgestrahlt und müssen daher zur Einhaltung von Emissionsgrenzwerten abgeschirmt werden.

Umgekehrt sind die für die Bildgebung zu empfangenden Magnetresonanzsignale extrem schwach. Um hier ein ausreichendes Signal-zu-Rausch-Verhältnis (SNR) zu erreichen, ist eine Abschirmung externer Störsignal erforderlich.

Deshalb werden im Stand der Technik um einen Magnetresonanztomographen aufwändige Schirmkabinen installiert, um sowohl Emissionen als auch Immissionen zu reduzieren.

Aus der Druckschrift DE 10 2014 207 843 ist eine Kniespule mit einer lokalen Schirmung bekannt.

Es könnte daher eine Aufgabe der Erfindung sein, den Aufwand für eine Abschirmung zu reduzieren.

Die Aufgabe wird durch einen erfindungsgemäßen Magnetresonanztomographen nach Anspruch 1 und ein erfindungsgemäßes Verfahren zum Betrieb des Magnetresonanztomographen nach Anspruch 10 gelöst.

Der erfindungsgemäße Magnetresonanztomograph weist einen Patiententunnel, eine erste Empfangsantenne zum Empfang eines Magnetresonanzsignals aus einem Patienten in dem Patiententunnel, eine zweite Empfangsantenne zum Empfang eines Signals mit der Larmorfrequenz des Magnetresonanzsignals und einen Empfänger auf. Der Empfänger steht mit der ersten Empfangsantenne und der zweiten Empfangsantenne in Signalverbindung und ist vorzugsweise ausgelegt, das Magnetresonanzsignal für eine Bildgebung aufzubereiten.

Die zweite Empfangsantenne ist außerhalb oder in der Nähe einer Öffnung des Patiententunnels angeordnet. Als Öffnung des Patiententunnels werden insbesondere die Öffnungen angesehen, durch die die Patientenliege mit dem Patienten in den Patiententunnel gefahren werden und, je nach zu untersuchendem Bereich, am gegenüberliegenden Ende den Patiententunnel auch wieder verlässt. In der Nähe wird dabei ein Abstand von der Öffnung von weniger als 0,1 m, 0,2 m. 0,5 m, 1 m oder 2 m angesehen. In der Nähe kann auch als ein Abstand von der Öffnung von weniger als einer viertel Wellenlänge oder einer halbe Wellenlänge einer Radiowelle in der Luft mit einer Larmorfrequenz des Magnetresonanztomographen angesehen werden.

Die erste Empfangsantenne empfängt dabei bevorzugt das Magnetresonanzsignal, das aber immer auch einen kleinen Anteil des Störsignals aufweist. Umgekehrt empfängt die zweite Empfangsantenne nicht nur das Störsignal, sondern auch einen minimalen bzw. vernachlässigbaren Anteil des Magnetresonanzsignals. Der Einfachheit halber wird dennoch im Folgenden das von der ersten Empfangsantenne empfangene Signal als Magnetresonanzsignal (wenn auch mit einem zu beseitigenden Anteil des störenden Signals) und das von der bzw. den zweiten Empfangsantennen empfangene Signal als Störsignal bezeichnet.

Der Empfänger ist dabei ausgelegt, ein mit der zweiten Empfangsantenne empfangenes Störsignal in einem von der ersten Empfangsantenne empfangenen Magnetresonanzsignal zu unterdrücken. Beispielhafte Ausführungsformen sind zu den Unteransprüchen näher beschrieben.

Beispielsweise kann der Empfänger eine Summationseinrichtung aufweisen, die eine von einem oder mehreren Parametern abhängige Linearkombination von Magnetresonanzsignal und Störsignal bildet. Weiterhin kann der Empfänger eine Entstörsteuerung aufweisen, die ausgelegt ist, den oder die Parameter so zu variieren, dass eine Energie des Störsignals in der Linearkombination minimal wird. Dabei können einer oder mehrere Parameter komplex sein, um eine Phasenverschiebung zu modellieren oder eine Phasenverschiebung durch einen eigenen Parameter angegeben sein. Mehrere Parameter erlauben insbesondere die effektive Unterdrückung unterschiedlicher Störquellen.

Der Empfänger kann dabei ausgelegt sein, diese erfindungsgemäße Verarbeitung des empfangenen Magnetresonanzsignals und des Störsignals zur Unterdrückung des Störsignals in Echtzeit auszuführen, beispielsweise mittels einer programmierbaren Logik (FPGA) oder eines Signalprozessors (DSP).

Es ist aber auch möglich, dass der Empfänger einen Speicher aufweist und das empfangene Störsignal und das empfangene Magnetresonanzsignal zunächst speichert, wobei die Unterdrückung des Störsignals erst zu einem späteren Zeitpunkt mit einer Verzögerung beispielsweise von der Dauer einer Echosequenz, einer Anregungssequenz oder einer ganzen Bilderfassung einer einzelnen Schicht oder der ganzen Bilderfassungssequenz erfolgt. Die Verzögerung kann beispielsweise größer als 50 ms, 100 ms, 0,5 s, 1 s, 10s 1 min oder mehr sein.

Als Empfänger im Sinne der Erfindung kann dabei die Hardware zur analogen und/oder digitalen Hochfrequenzverarbeitung wie beispielsweise Verstärker, Filter und Mischer in Echtzeit, aber auch eine Bildauswerteeinheit zur späteren Erzeugung einer Abbildung aus den empfangenen Magnetresonanzsignalen angesehen werden.

Das erfindungsgemäße Verfahren zum Betrieb des erfindungsgemäßen Magnetresonanztomographen weist den Schritt auf, mittels des Empfängers Störsignals durch über die zweite Empfangsantenne zu empfangen. Aufgrund der Anordnung der zweiten Antenne in der Nähe der Öffnung weist das von der zweiten Antenne empfangene Störsignal kaum oder gar keine Anteile des Magnetresonanzsignals auf.

In einem anderen Schritt empfängt der Empfänger ein Magnetresonanzsignal über die erste Empfangsantenne. Bei der ersten Empfangsantenne kann es sich beispielsweise um eine Körperspule oder eine Lokalspule des Magnetresonanztomographen handeln.

In einem weiteren Schritt des Verfahrens verarbeitet der Empfänger das Magnetresonanzsignal in Abhängigkeit von dem Störsignal durch den Empfänger zu einem Empfangssignal, wobei die Abhängigkeit von einem Parameter abhängt. Beispielsweise ist es denkbar, dass der Empfänger eine Linearkombination aus Störsignal und Magnetresonanzsignal mit dem Parameter als Faktor bildet, wobei der Parameter komplex sein kann, um eine Phasenverschiebung abzubilden. Auch sind mehrere Parameter denkbar.

In einem anderen Schritt stellt der Empfänger, beispielsweise mittels einer Entstörsteuerung, den Parameter so ein, dass ein Anteil des Störsignals in dem Empfangssignal reduziert wird. Denkbar ist es beispielsweise, dass der Parameter durch die Entstörsteuerung in einem Optimierungsverfahren so eingestellt wir, dass eine Energie des Störsignals in dem Empfangssignal minimiert wird.

Wie bereits zuvor zum Empfänger erläutert, kann der Empfänger dabei das Magnetresonanzsignal und/oder das Störsignal auch speichern, sodass die Schritte des Verarbeitens und des Einstellens des Parameters auch mit zeitlichem Abstand zum Empfangen der Signale durch die erste Empfangsantenne und die zweite Empfangsantenne erfolgen können.

Auf vorteilhafte Weise ermöglicht es der erfindungsgemäße Magnetresonanztomograph und das Verfahren zum Betrieb durch die zweite Empfangsantenne und den erfindungsgemäßen Empfänger, den Anteil externer Störsignale in dem Magnetresonanzsignal zu reduzieren und daher mit einfacheren und kostengünstigeren Abschirmmaßnahmen auszukommen.

Weitere vorteilhafte Ausführungsformen sind in den Unteransprüchen angegeben.

In einer möglichen Ausführungsform des erfindungsgemäßen Magnetresonanztomographen ist der Magnetresonanztomograph ausgelegt, Magnetresonanzsignale mit einer Larmorfrequenz in einem Industrieband zu empfangen. Als Larmorfrequenz des Magnetresonanztomographen wird dabei eine Resonanzfrequenz der für die Bildgebung in dem Magnetresonanztomographen genutzten Kernspins in einem statischen Magnetfeld B0 eines Feldmagneten des Magnetresonanztomographen bezeichnet. Als Industrieband werden für eine Nutzung durch medizinische oder technische Geräte freigegebene Frequenzbänder bezeichnet, für die erleichterte Vorschriften bei der Emission und Genehmigung vorliegen. Diese werden auch als ISM-Bänder (Industrial, Scientific, Medical Band) bezeichnet. Ein beispielhaftes Frequenzband, in dem auch mit großen Leistungen emittiert werden darf, liegt zwischen 26.9 und 27,3 MHz. Andere derartige Frequenzbänder liegen zwischen 6,7 MHz und 6,8 MHz, 13,5 MHz und 13,6 MHz, 40,6 MHz und 40,7 MHz sowie 433,0 MHz und 434,8 MHz.

Ein Magnetresonanztomograph ist nicht nur darauf angewiesen, dass möglichst geringe Störungen empfangen werden, sondern die starken Anregungsimpulse dürfen auch andere Geräte nicht stören. Im ISM-Band sind die gesetzlichen Toleranzgrenzen wesentlich höher, sodass eine gesetzeskonforme Begrenzung durch Schirmung der emittierten Anregungspulse bei dieser Frequenz einfacher möglich ist oder auch nicht erforderlich ist. In synergistischer Verbindung mit der aktiven Schirmung, um empfangsseitig Störungen durch andere Geräte im ISM-Band zu unterdrücken, kann so auf vorteilhafte Weise ein Magnetresonanztomograph ganz ohne Schirmkabine realisiert werden.

In einer denkbaren Ausführungsform des erfindungsgemäßen Magnetresonanztomographen ist eine Grenzfrequenz für eine Ausbreitung einer Radiowelle in dem Patiententunnel größer als eine Larmorfrequenz des Magnetresonanztomographen. Als Grenzfrequenz wird die Frequenz angesehen, bei der sich in dem Patiententunnel als Hohlleiter noch eine sich in Längs-Richtung (z-Richtung) durch den Patiententunnel ausbreitende Radiowelle ausbilden kann.

Liegt die Frequenz eines Radiosignals darunter, so erfolgt auf vorteilhafte Weise ein exponentieller Abfall der Feldstärke mit dem Abstand von der Öffnung im Inneren des Patiententunnels, sodass im Untersuchungsbereich (Field of View, FoV) das Störsignal wesentlich reduziert ist.

In einer möglichen Ausführungsform des erfindungsgemäßen Magnetresonanztomographen ist die zweite Empfangsantenne an einer Öffnung des Patiententunnels oder der Patientenliege angeordnet. Beispielsweise kann die zweite Empfangsantenne unmittelbar an einem Rand einer Öffnung angeordnet sein oder unter der Liegefläche für den Patienten.

Insbesondere, wenn die Frequenz des Störsignals unter der Grenzfrequenz für eine freie Welle liegt, bilden der Patient und der Patiententunnel einen Koaxialleiter für das Störsignal aus. Eine zweite Empfangsantenne in der Nähe des Patienten und der Öffnung kann auf vorteilhafte Weise das durch den Patienten in den Patiententunnel eingekoppelte Störsignal erfassen und so eine besonders effektive Unterdrückung durch den Empfänger ermöglichen.

In einer denkbaren Ausführungsform des erfindungsgemäßen Magnetresonanztomographen weist die zweite Empfangsantenne eine im Wesentlichen Rundum-Empfangscharakteristik auf. Als im Wesentlichen Rundum-Empfangscharakteristik wird eine Empfindlichkeitsverteilung der Empfangsantenne in alle Raumrichtungen angesehen, bei der die Unterschied zwischen einer maximalen Empfindlichkeit und einer minimalen Empfindlichkeit in Abhängigkeit von den unterschiedlichen Richtungen kleiner als 6 dB, 12 dB, 18 dB oder 24 dB ist. Dies gilt vorzugsweise auch für unterschiedliche Polarisationen des Störsignals

Das Störsignal kann aus unterschiedlichen Richtungen und mit unterschiedlichen Polarisationen einfallen, sodass eine Antenne mit Richtcharakteristik oder Vorzugspolarisation nicht alle Störsignale erfassen kann. Eine Antenne mit Rundum-Empfangscharakteristik hingegen kann auf vorteilhafte Weise alle Störsignale erfassen.

In einer möglichen Ausführungsform des erfindungsgemäßen Magnetresonanztomographen weist der Magnetresonanztomograph eine Mehrzahl an zweiten Antennen auf und der Empfänger ist ausgelegt, das Störsignal in dem Magnetresonanzsignal in Abhängigkeit von Empfangssignalen der Mehrzahl der zweiten Empfangsantenne zu unterdrücken. Vorzugsweise sind die Mehrzahl der zweiten Empfangsantennen zueinander beabstandet angeordnet, beispielsweise in einem Abstand größer als ein Viertel einer Wellenlänge oder einer halben Wellenlänge einer Radiowelle mit der Larmorfrequenz.

Auch eine Mehrzahl an räumlich verteilten Antennen ist auf vorteilhafte Weise geeignet, eine bzw. mehrere Störquellen besser zu erfassen und damit die Störunterdrückung zu verbessern.

In einer denkbaren Ausführungsform des erfindungsgemäßen Magnetresonanztomographen ist die Mehrzahl der Empfangsantennen in einer Symmetrieanordnung zu dem Patiententunnel angeordnet. Denkbar ist beispielsweise eine Anordnung an dem Rand der Öffnung des Patiententunnels an zwei gegenüberliegenden Punkten, auf den Eckpunkten eines regelmäßigen Polygons oder Polyeders.

Auf vorteilhafte Weise kann mittels der Symmetrie der Antennen auch eine Symmetriebeziehung der Parameter hergestellt und so das Optimierungsverfahren zur Reduzierung des Störsignals vereinfacht und/oder beschleunigt werden.

In einer möglichen Ausführungsform des erfindungsgemäßen Verfahrens erfolgt der Schritt des Einstellens in einem Zeitraum einer Sequenz, in der kein Magnetresonanzsignal empfangen wird, insbesondere auch kein Signal zur Anregung der Spins gesendet wird. Innerhalb einer Sequenz zur Bilderfassungen gibt es Zeiträume, in denen keine Anregungspulse emittiert werden und auch keine Erfassung eines Magnetresonanzsignals für eine Bildgebung erfolgt. Vorzugsweise handelt es sich dabei auch um Zeiträume der Sequenz, in der das Untersuchungsobjekt bzw. der Patient kein nennenswertes Magnetresonanzsignal emittiert, d.h. das Signal ist im Pegel mindestens 12 dB, 24 dB, 36 dB 48 dB oder 60 dB unter einem maximalen Magnetresonanzsignal. In einem derartigen Zeitraum erfolgt in dieser Ausführungsform des Verfahrens der Schritt des Einstellens.

Die Entstörsteuerung des Empfängers ist entsprechend ausgelegt, den Schritt des Einstellens in einem derartigen Zeitraum auszuführen. Beispielsweise kann Sie ein Triggersignal von der Steuerung des Magnetresonanztomographen erhalten.

Auf vorteilhafte Weise kann ohne Magnetresonanzsignal das Einstellen der Parameter vereinfacht werden, beispielsweise indem eine Energie des von der ersten Empfangsantenne empfangenen Signals in Abhängigkeit von den Parametern minimiert wird. Selbst wenn das Störsignal sich zeitlich ändert, so sind doch die eingestellten Parameter bei gleicher räumlicher Anordnung weiterhin gültig und wirksam.

In einer anderen möglichen Ausführungsform des erfindungsgemäßen Verfahrens ist es aber auch denkbar, dass die Einstellung des Parameters permanent, d.h. in kurzen Abständen von 1 ms, 10 ms oder 100 ms erfolgt oder insbesondere in Echtzeit mit einer Verzögerung von weniger als 10, 100 oder 500 Mikrosekunden erfolgt.

Auf vorteilhafte Weise erlaubt es die permanente Einstellung des Parameters in Echtzeit oder nahezu Echtzeit, schnell auf neu auftretende Störsignale zu reagieren und deren negativen Effekt auf die Bildgebung möglichst zu minimieren.

In einer weiteren denkbaren Ausführungsform weist der Empfänger einen Speicher auf und speichert das empfangene Magnetresonanzsignal und das empfangene Störsignal. Der Empfänger kann dabei im Sinne der Erfindung beispielsweise auch einen Bildauswerterechner umfassen. Es ist in einer Ausführungsform aber auch denkbar, dass der Empfänger im engeren Sinne, das heißt die zur Aufbereitung der empfangenen hochfrequenten Magnetresonanzsignale genutzten Einrichtungen, den Speicher aufweisen. Das Einstellen des Parameters und das Verarbeiten des Magnetresonanzsignals mit dem Störsignal in Abhängigkeit von dem Parameter zur Reduzierung des Störsignals durch den Empfänger zu einem Empfangssignal kann dann mit einer Verzögerung zu dem Empfang erfolgen. Die Verzögerung kann beispielsweise die Dauer einer Echosequenz, einer Anregungssequenz oder auch einer ganzen Bilderfassungssequenz umfassen, beispielsweise mehr als 10 ms, 100 ms, 0,5 s 10 s oder auch mehrerer Minuten Stunden oder prinzipiell auch Tage.

Auf vorteilhafte Weise ist es durch das Speichern möglich, bereits vorhandene Ressourcen des Magnetresonanztomographen mit zu nutzen oder wegen der nicht erforderlichen Echtzeitbearbeitung auch mit geringerer Rechenleistung die Störunterdrückung kostengünstiger bereitzustellen. Auch ermöglicht die nachträgliche Störunterdrückung einen Vergleich unterschiedlicher Parametereinstellungen und Unterdrückungsverfahren.

In einer denkbaren Ausführungsform des erfindungsgemäßen Verfahrens weist der Empfänger bzw. die Entstörsteuerung eine Autokorrelationsvorrichtung auf. Mittels der Autokorrelationsvorrichtung kann die Entstörsteuerung einen Anteil des Störsignals in dem Magnetresonanzsignal bestimmen, beispielsweise eine Amplitude und eine Phasenverschiebung.

In einer anderen möglichen Ausführungsform weist die Entstörsteuerung eine Schätzeinrichtung auf, die den Anteil des Störsignals beispielsweise durch ein Optimierungsverfahren ermittelt, bei dem der Störanteil im Magnetresonanzsignal durch Variation des oder der Parameter minimiert wird, wie Least Mean Square Root oder ähnliche Verfahren.

Bei mehreren Parametern optimiert die Entstörsteuerung die Mehrzahl der Parameter so, dass ein möglichst großer Anteil des Störsignals reduziert wird. Dies kann insbesondere bei mehreren Störquellen oder Reflexionen von Vorteil sein.

Auf vorteilhafte Weise erlauben Autokorrelation oder Schätzung eine flexible Anpassung an unterschiedliche Störquellen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:

Fig. 1   eine schematische Darstellung eines erfindungsgemäßen Systems aus Magnetresonanztomograph, Empfangsantenne und Anschlussleitung.

Fig. 2   eine schematische Darstellung des Empfängers und der ersten Empfangsantenne und der zweiten Empfangsantennen;

Fig. 3   eine schematische Darstellung eines Ablaufplans einer Ausführungsform des erfindungsgemäßen Verfahrens.

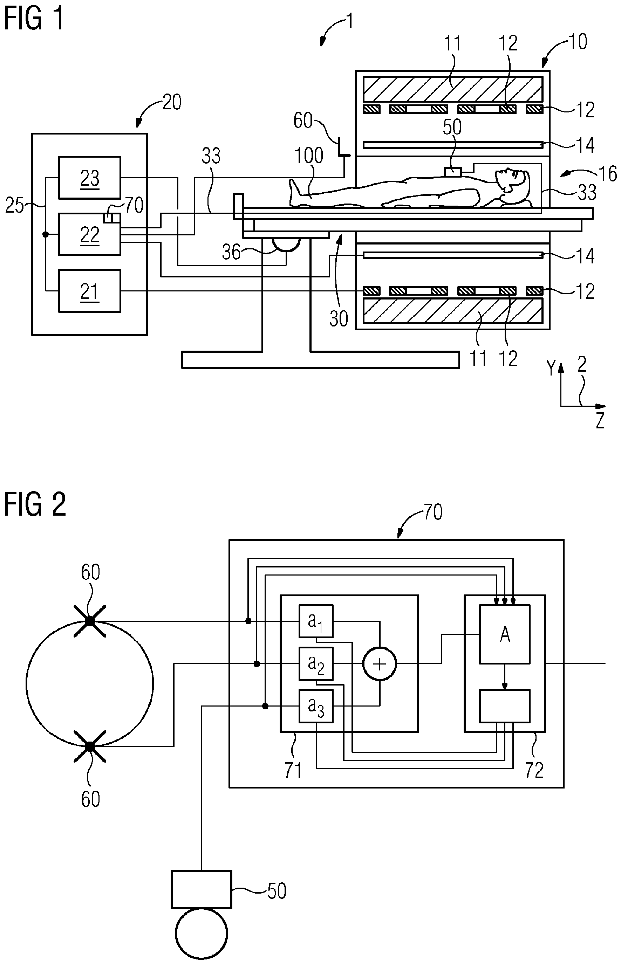

Fig. 1 zeigt eine schematische Darstellung einer Ausführungsform eines Magnetresonanztomographen 1 mit einer erfindungsgemäßen Lokalspule 50.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungspulse können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Auf dem Patienten 100 ist als eine erste Empfangsspule eine Lokalspule 50 angeordnet, die über eine erfindungsgemäße Anschlussleitung 33 mit der Hochfrequenzeinheit 22 und deren Empfänger verbunden ist. Denkbar ist es aber auch, dass die Körperspule 14 eine erste Empfangsantenne im Sinne der Erfindung ist.

An einem Rand der Öffnung des Patiententunnels 16 sind vier zweite Empfangsantennen 60 angeordnet, die an den Ecken eines Quadrates angeordnet sind, das der kreisförmigen Öffnung einbeschrieben ist, sodass die Ecken auf dem Rand der Öffnung zu liegen kommen. Die vier zweiten Empfangsantennen 60 stehen in Signalverbindung mit dem Empfänger 70 der Hochfrequenzeinheit 22. Aufgrund der Mehrzahl der zweiten Empfangsantennen 60 ist es dabei denkbar, dass diese nicht alle eine Rundum-Empfangscharakteristik aufweisen, sondern beispielsweise Dipole sind und sich durch die unterschiedliche Ausrichtung zu einer Rundum-Charakteristik ergänzen. Es wäre aber beispielsweise auch denkbar, als einzige zweite Antenne mit Rundum-Charakteristik einen Kreuzdipol vorzusehen.

Es ist auch möglich, dass alternativ oder zusätzlich eine zweite Empfangsantenne 60 in der Patientenliege 30 angeordnet ist.

Der Patiententunnel hat dabei vorzugsweise einen Radius R für den gilt:

$$R < (Lambda_L * 1,841)/(2 * Pi)$$

$Lambda_L$ gibt dabei die Wellenlänge einer Radiowelle in Luft bei der Larmorfrequenz des Magnetresonanztomographen 1 an. Ist der Radius R kleiner als der rechte Term, so breitet sich die Radiowelle in dem Patiententunnel 16 exponentiell gedämpft aus und das Störsignal ist in der Mitte im Untersuchungsbereich FoV stark gedämpft. $Lambda_L$ wird auch als Grenzwellenlänge eines Rundhohlleiters bezeichnet, die dazugehörige Frequenz als Grenzfrequenz.

Lediglich der Patient 100 wirkt durch seine endliche Leitfähigkeit als Seele eines Koaxialkabels, dessen Mantel die Wand des Patiententunnels 16 ist, und leitet ein bei den Beinen oder dem Kopfende eingekoppeltes elektromagnetisches Signal in den Untersuchungsbereich weiter. Die zweite Empfangsantenne 70 bzw. die zweiten Empfangsantennen 70 in der Nähe der Öffnung oder in der Patientenliege 30 nehmen dabei auf vorteilhafte Weise das von dem Patienten 100 in den FoV weitergeleitete Störsignal auf und machen dadurch die Kompensation in dem Empfänger 70 besonders effektiv.

Fig. 2 gibt eine schematische Darstellung der Funktionseinheiten einer möglichen Ausführungsform eines Empfängers 70 dar.

Die Summationseinrichtung gewichtet die von der ersten Empfangsantennen bzw. Lokalspule 50 und von den zweiten Empfangsantennen eingehenden Signal mit Parametern, die auch komplex sein können, um eine Phasenverschiebung anzugeben. In einem analogen Empfänger 70 kann dies durch einen einstellbaren Verstärker in Verbindung mit einem einstellbaren Phasenschieber erfolgen. Der Realteil des Parameters entspricht dann dem Verstärkungsfaktor und der Imaginärteil der Phasenverschiebung. Anschließend werden in einer bevorzugten Ausführungsform die gewichteten Signale summiert, es sind aber auch andere nichtlineare Signaloperationen zur Kombination der Einzelsignale denkbar.

Eine Entstörsteuerung 72 erhält das kombinierte Signal als auch die einzelnen Signale der ersten Empfangsantenne und der zweiten Empfangsantennen 60. Um einen Anteil des Störsignals in dem kombinierten Signal zu bestimmen, ist es beispielsweise denkbar, dass die Entstörsteuerung 72 eine Autokorrelation der Signale vornimmt. Es ist aber auch denkbar, dass die Energie des kombinierten Signals bestimmt wird. In einer denkbaren Ausführungsform bestimmt die Entstörsteuerung 72 den Anteil des Störsignals in Abschnitten von Sequenzen des Magnetresonanztomographen, in denen kein Magnetresonanzsignal zur Bildgebung erwartet wird, sodass das kombinierte Signal nur das Störsignal aufweist. Dies kann beispielsweise in dephasierten Abschnitten einer Echosequenz der Fall sein, da sich die Amplituden einzelner Kernspins aufgrund unterschiedlicher Phase aufheben und in Summe kein Signal erzeugen.

Die Entstörsteuerung 72 optimiert dann beispielsweise nach einem Least-Square-Root-Verfahren (LSR) die Parameter in der Summationseinrichtung, sodass der Anteil bzw. die Energie des Störsignals in dem kombinierten Signal minimiert wird.

Grundsätzlich kann der Empfänger 70 sowohl in analoger Signalverarbeitungstechnik ausgeführt sein, sodass z.B. Verstärkungsregelung und Phasenverschiebung durch einen Parameter gesteuert und dann analog umgesetzt werden, oder auch als digitaler Empfänger, der entweder bereits digitalisierte Signale von der ersten Empfangsantenne und/ oder der zweiten Empfangsantenne 60 erhält oder diese bereits am Signaleingang mittels eines A/D-Wandlers digitalisiert.

Der Empfänger 70 leitet das kombinierte Signal, in dem das Störsignal weitestgehend unterdrückt ist, zur Bildgebung an die Steuerung 23 des Magnetresonanztomographen weiter.

In einer anderen denkbaren Ausführungsform des erfindungsgemäßen Magnetresonanztomographen (1) erfolgen die Unterdrückung des Störsignals in dem Empfangssignal nicht in Echtzeit, d.h. nicht unmittelbar bei Empfang des Störsignals und/oder des Magnetresonanzsignals, sondern das Magnetresonanzsignal und das Störsignal werden von dem Empfänger 70, der dabei auch Teile der Steuerung 23 des Magnetresonanztomographen 1 oder einer Bildauswertung umfassen kann, in einem Speicher gespeichert. Die nachfolgend zu dem Verfahren dargelegten Schritte erfolgen dann nicht mehr in Echtzeit oder nahezu in Echtzeit, sondern können auf den gespeicherten Daten mit Verzögerung ausgeführt werden, beispielsweise im Vorfeld einer Bildauswertung.

Die in Fig. 2 dargestellt Störunterdrückung in dem Empfänger kann auch mit einer einzelnen zweiten Antenne 60 ausgeführt werden. Umgekehrt ist es möglich, dass der Empfänger 70 mehrere Kanäle aufweist oder mehrere Empfänger 70 in dem Magnetresonanztomographen 1 vorgesehen sind, um die Magnetresonanzsignale mehrerer Lokalspulen 50 zu entstören. Dabei ist es denkbar, dass dabei die Signale der zweiten Empfangsantennen 60 von mehreren Empfängern 70 bzw. Kanälen der Empfänger 70 zur Störunterdrückung genutzt werden.

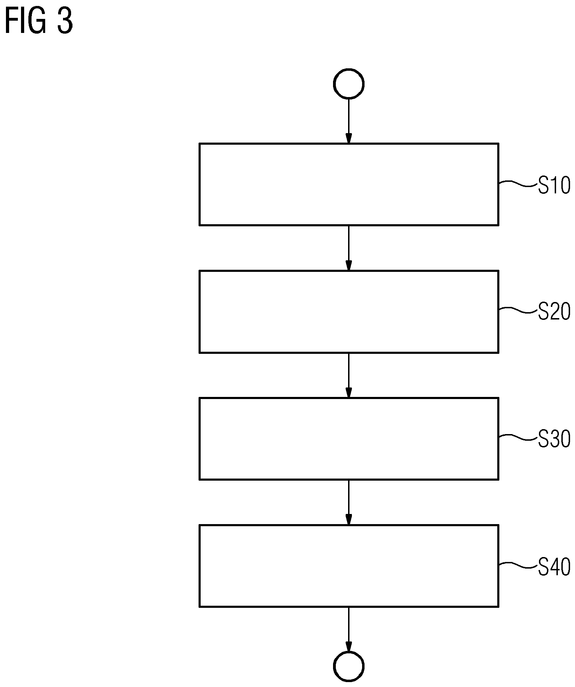

Fig. 3 zeigt einen schematischen Ablaufplan eines erfindungsgemäßen Verfahrens.

In einem Schritt S10 empfängt der Empfänger 70 ein Störsignal über die zweite Empfangsantenne 60 bzw. über die Mehrzahl an zweiten Antennen 60. Das Störsignal wird über eine Signalverbindung zu dem Empfänger 70 übertragen werden. Dabei ist es auch denkbar, dass das Störsignal zunächst durch einen A/D-Wandler digitalisiert wird, bevor es zu dem Empfänger 70, in diesem Fall als digitaler Empfänger 70 ausgeführt, übertragen wird.

In einem Schritt S20 empfängt der Empfänger 70 ein Magnetresonanzsignal über die erste Empfangsantenne, beispielsweise die Lokalspule 50. Bei mehreren Lokalspulen 50 können entsprechend mehrere Empfänger 70 vorgesehen sein oder auch ein Empfänger 70 mit mehreren Kanälen mit jeweils einer Summationseinrichtung 71. Die Entstörsteuerung 72 kann dabei jeweils separat vorgesehen sein oder auch gemeinsam, was das nachfolgende Einstellen wegen ähnlicher Parameter für unterschiedliche Kanäle beschleunigen kann.

In einem Schritt S30 verarbeitet der Empfänger 70 das Magnetresonanzsignal in Abhängigkeit von dem Störsignal bzw. den Störsignalen bei mehreren zweiten Empfangsantennen 60 zu einem Empfangssignal. Beispielsweise werden das bzw. die Störsignale der zweiten Empfangsantenne bzw. zweiten Empfangsantennen 60 und das Magnetresonanzsignal der ersten Empfangsantenne mit unterschiedlichen Parametern gewichtet und verzögert und anschließend kombiniert. Das kann beispielsweise die Erzeugung einer Linearkombination sein. Das erzeugte Empfangs- bzw. Summensignal hängt dabei von einem bzw. mehreren Parametern ab.

In einem anderen Schritt S40 wird der Parameter bzw. die Parameter durch den Empfänger 70, insbesondere die Entstörsteuerung 72 derart eingestellt, dass ein Anteil des Störsignals in dem Empfangssignal reduziert wird. Wird beispielsweise das von der zweiten Empfangsantenne 60 empfangene Störsignal durch den eingestellten Parameter so skaliert, dass es gleiche Amplitude wie der über die erste Empfangsantenne Störsignalanteil aufweist und wird es mit einer Phasenverschiebung relativ dazu von 180 Grad versehen, so hebt sich das Störsignal in dem erzeugten Empfangssignal genau auf. Der Parameter bzw. die Parameter können dabei über Optimierungsverfahren wie beispielsweise Least Square Root (LSR) oder Wiener Filter ermittelt werden.

Es ist dabei auch denkbar, dass die Schritte S10 bis S30 jeweils auf empfangene Magnetresonanzsignale und empfangene Störsignale in Echtzeit ausgeführt werden, insbesondere bei analogen Empfängern 70. Es ist aber auch denkbar, dass die Schritte S10 bis S30 jeweils auf gespeicherte Störsignale und Magnetresonanzsignale ausgeführt werden, die beispielsweise für eine einzelne Sequenz oder einzelne Abschnitte davon digitalisiert werden.

In einer möglichen Ausführungsform des erfindungsgemäßen Verfahrens wird der Schritt S40 mit Störsignalen aus einem Zeitraum einer Sequenz ausgeführt, in der kein Magnetresonanzsignal zur Bildgebung empfangen wird. Beispielsweise können die Parameter mit Störsignalen der zweiten Empfangsantenne 60 und einem Signal der ersten Empfangsantenne in einer Phase von der Entstörsteuerung 72 bestimmt werden, in der die Kernspins dephasiert sind und kein Magnetresonanzsignal erzeugen. Es ist aber auch denkbar, dass in diesem Zeitraum ohne Magnetresonanzsignal das Störsignal und das Signal der ersten Empfangsantenne lediglich digital erfasst und später ausgewertet wird.

Grundsätzlich ist es in einer Ausführungsform des erfindungsgemäßen Verfahrens auch denkbar, dass das empfangene Störsignal und/oder Magnetresonanzsignal von dem Empfänger 70 in einem Schritt S25 gespeichert wird. Der Empfänger 70 kann dabei auch Teile der Steuerung 23 des Magnetresonanztomographen 1 oder eines externen Bildauswerterechners umfassen. Die Schritte S20 bis S40 werden dabei nachträglich ausgeführt, beispielsweise am Ende einer Echosequenz, einer Anregungssequenz, einer Signalerfassung für eine Schicht des Untersuchungsobjektes oder auch nach Erfassung aller Daten.

Die Entkopplung der Signalerfassung von Störsignal und Magnetresonanzsignal von der Störunterdrückung ermöglichen es so auf vorteilhafte Weise, kostengünstigere Komponenten mit geringerer Rechenleistung einzusetzen oder auch vorhandene Ressourcen, z.B. aus der Bildauswertung doppelt zu nutzen. Auch ist es dann denkbar, unterschiedliche Parametereinstellungen zu vergleichen und auszuwählen oder nachträglich zu optimieren. Auch kann die Anwendung auf Zeiträume mit Störungen begrenzt werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

**1.** Magnetresonanztomograph, wobei der Magnetresonanztomograph (1) einen Patiententunnel (16), eine erste Empfangsantenne zum Empfang eines Magnetresonanzsignals aus einem Patienten (100) in dem Patiententunnel (16), eine zweite Empfangsantenne (60) zum Empfang eines Signals mit der Larmorfrequenz des Magnetresonanzsignals, und einen Empfänger (70) aufweist, wobei die zweite Empfangsantenne (60) außerhalb oder in der Nähe einer Öffnung des Patiententunnels (16) angeordnet ist, wobei der Empfänger (70) in Signalverbindung mit der ersten Empfangsantenne und der zweiten Empfangsantenne (60) steht und der Empfänger (70) ausgelegt ist, ein mit der zweiten Empfangsantenne (60) empfangenes Störsignal in einem von der ersten Empfangsantenne empfangenen Magnetresonanzsignal zu unterdrücken.

2. Magnetresonanztomograph nach Anspruch 1, wobei der Magnetresonanztomograph (1) ausgelegt ist, Magnetresonanzsignale mit einer Larmorfrequenz in einem Industrieband zu empfangen.

3. Magnetresonanztomograph nach Anspruch 1 oder 2, wobei eine Grenzfrequenz für eine Ausbreitung einer Radiowelle in dem Patiententunnel (16) größer ist als eine Larmorfrequenz des Magnetresonanztomographen (1).

4. Magnetresonanztomograph nach einem der Ansprüche 1 bis 3, wobei die zweite Empfangsantenne (60) an einer Öffnung des Patiententunnels (16) oder der Patientenliege (30) angeordnet ist.

5. Magnetresonanztomograph nach einem der vorhergehenden Ansprüche, wobei die zweite Empfangsantenne (60) eine Rundum-Empfangscharakteristik aufweist.

6. Magnetresonanztomograph nach einem der vorhergehenden Ansprüche, wobei der Magnetresonanztomograph (1) eine Mehrzahl an zweiten Empfangsantennen (60) aufweist und der Empfänger (70) ausgelegt ist, das Störsignal in dem Magnetresonanzsignal in Abhängigkeit von Empfangssignalen der Mehrzahl der zweiten Empfangsantennen (60) zu unterdrücken.

7. Magnetresonanztomograph nach Anspruch 6, wobei die Mehrzahl der zweiten Empfangsantennen (60) in einer Symmetrieanordnung zu dem Patiententunnel (16) angeordnet ist.

8. Magnetresonanztomograph nach einem der vorhergehenden Ansprüche, wobei der Empfänger (70) eine Autokorrelationseinrichtung aufweist und die Autokorrelationseinrichtung ausgelegt ist, einen Anteil des von der zweiten Empfangsantenne (60) empfangenen Signals in dem von der ersten Empfangsantenne empfangenen Magnetresonanzsignal zu bestimmen.

9. Magnetresonanztomograph nach einem der Ansprüche 1 bis 7, wobei der Empfänger (70) eine Schätzeinrichtung aufweist und die Schätzeinrichtung ausgelegt ist, einen Anteil des von der zweiten Empfangsantenne (60) empfangenen Signals in dem von der ersten Empfangsantenne empfangenen Magnetresonanzsignal zu schätzen.

10. Verfahren zum Betrieb eines Magnetresonanztomographen (1), wobei der Magnetresonanztomograph (1) einen Patiententunnel (16), eine erste Empfangsantenne zum Empfang eines Magnetresonanzsignals aus einem Patienten (100) in dem Patiententunnel (16), eine zweite Empfangsantenne (60) zum Empfang eines Signals mit der Larmorfrequenz des Magnetresonanzsignals und einen Empfänger (70) aufweist, wobei die zweite Empfangsantenne (60) außerhalb des Patiententunnels (16) oder in der Nähe einer Öffnung des Patiententunnels (16) angeordnet ist und wobei das Verfahren die Schritte aufweist:

    (S10) Empfangen eines Störsignals durch den Empfänger (70) über die zweite Empfangsantenne (60);
    (S20) Empfangen eines Magnetresonanzsignals durch den Empfänger (70) über die erste Empfangsantenne;
    (S30) Verarbeiten des Magnetresonanzsignals in Abhängigkeit von dem Störsignal durch den Empfänger (70) zu einem Empfangssignal, wobei die Abhängigkeit von einem Parameter abhängt;
    (S40) Einstellen des Parameters durch den Empfänger (70), sodass ein Anteil des Störsignals in dem Empfangssignal reduziert wird.

11. Verfahren nach Anspruch 10, wobei der Schritt (S40) des Einstellens mit Störsignalen aus einem Zeitraum einer Sequenz erfolgt, in der kein Magnetresonanzsignal zur Bildgebung empfangen wird.

12. Verfahren nach Anspruch 10 oder 11, wobei der Empfänger (70) einen Speicher aufweist und das Verfahren einen Schritt (S25) des Speicherns aufweist, bei dem der Empfänger (70) das Störsignal sowie das Magnetresonanzsignal in dem Speicher speichert, wobei der Schritt (S30) des Verarbeitens mit einer Verzögerung relativ zu dem Empfangen des Störsignals und/oder Magnetresonanzsignals erfolgt.

13. Verfahren nach Ansprüche 10 bis 12, wobei der Empfänger (70) eine Autokorrelationseinrichtung aufweist und in dem Schritt (S40) des Einstellens des Parameters die Autokorrelationseinrichtung einen Anteil des Störsignals in dem Magnetresonanzsignal bestimmt und der Parameter in Abhängigkeit von dem bestimmten Anteil des Störsignals eingestellt wird.

14. Verfahren nach Anspruch 10 bis 12, wobei der Empfänger (70) eine Schätzeinrichtung aufweist und in dem Schritt (S40) des Einstellens des Parameters die Schätzeinrichtung einen Anteil des Störsignals in dem Magnetresonanzsignal bestimmt und der Parameter in Abhängigkeit von dem bestimmten Anteil des Störsignals eingestellt wird.

15. Computerprogrammprodukt, welches direkt in einen Prozessor einer programmierbaren Steuerung (23) ladbar ist, mit Programmcode-Mitteln, um alle Schritte eines Verfahrens nach einem der Ansprüche 10 bis 14 auszuführen, wenn das Programmprodukt auf der Steuerung (23) ausgeführt wird.

16. Computerlesbares Speichermedium mit darauf gespeicherten elektronisch lesbaren Steuerinformationen, welche derart ausgestaltet sind, dass sie bei Verwendung des Speichermediums in einer Steuerung (23) eines Magnetresonanztomographen (1) nach Anspruch 1 bis 9 das Verfahren nach einem der Ansprüche 10 bis 14 durchführen.

FIG 1

FIG 2

FIG 3

**Europäisches Patentamt**
**European Patent Office**
**Office européen des brevets**

## EUROPÄISCHER RECHERCHENBERICHT

Nummer der Anmeldung

EP 17 19 4969

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | WO 2015/150236 A1 (KONINKL PHILIPS NV [NL]) 8. Oktober 2015 (2015-10-08)<br>* Seite 14, Zeile 28 - Seite 24, Zeile 24 *<br>* Abbildung 1 *<br>----- | 1-4,6-16<br>5 | INV.<br>G01R33/36<br>G01R33/565<br><br>ADD.<br>G01R33/422 |
| X<br>Y | US 2008/048658 A1 (HUSHEK STEPHEN GERARD [US] ET AL) 28. Februar 2008 (2008-02-28)<br>* Absatz [0007] - Absatz [0053] *<br>* Abbildung 1 *<br>----- | 1-4,6-16<br>5 | |
| Y | WO 2013/016639 A1 (BRIGHAM & WOMENS HOSPITAL [US]; PATZ SAMUEL [US]; HROVAT MIRKO [US]; B)<br>31. Januar 2013 (2013-01-31)<br>* Absatz [0098] - Absatz [0107] *<br>* Abbildung 8 *<br>----- | 5 | |

**RECHERCHIERTE SACHGEBIETE (IPC)**

G01R
G01N
G01V
A61B

Der vorliegende Recherchenbericht wurde für alle Patentansprüche erstellt

| Recherchenort | Abschlußdatum der Recherche | Prüfer |
|---|---|---|
| München | 11. April 2018 | Streif, Jörg Ulrich |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 19 4969

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-04-2018

| Im Recherchenbericht angeführtes Patentdokument | Datum der Veröffentlichung | Mitglied(er) der Patentfamilie | Datum der Veröffentlichung |
|---|---|---|---|
| WO 2015150236 A1 | 08-10-2015 | CN 106164694 A<br>EP 3126861 A1<br>JP 2017515532 A<br>US 2017108569 A1<br>WO 2015150236 A1 | 23-11-2016<br>08-02-2017<br>15-06-2017<br>20-04-2017<br>08-10-2015 |
| US 2008048658 A1 | 28-02-2008 | US 2008048658 A1<br>WO 2008022441 A1 | 28-02-2008<br>28-02-2008 |
| WO 2013016639 A1 | 31-01-2013 | CA 2843422 A1<br>EP 2736409 A1<br>JP 2014523795 A<br>KR 20140063649 A<br>US 2014155732 A1<br>WO 2013016639 A1 | 31-01-2013<br>04-06-2014<br>18-09-2014<br>27-05-2014<br>05-06-2014<br>31-01-2013 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014207843